Europäisches Patentamt

European Patent Office

Office européen des brevets

(19)

(11) Publication number: **0 137 688**
**A2**

(12) **EUROPEAN PATENT APPLICATION**

(21) Application number: **84305844.7**

(22) Date of filing: **28.08.84**

(51) Int. Cl.⁴: **C 12 M 1/40**

(30) Priority: **31.08.83 GB 8323358**

(43) Date of publication of application:
**17.04.85 Bulletin 85/16**

(84) Designated Contracting States:
**AT BE CH DE FR GB IT LI LU NL SE**

(71) Applicant: **IMPERIAL CHEMICAL INDUSTRIES PLC**
**Imperial Chemical House Millbank**
**London SW1P 3JF(GB)**

(72) Inventor: **Hines, David Albert**
**62 Church Road**
**Hale Village Liverpool L24 4BA(US)**

(72) Inventor: **Middleton, John Colin**
**109 Mount Pleasant Road**
**Davenham Northwich Cheshire(GB)**

(72) Inventor: **Steele, Margaret Lilian**
**21 Bunbury Drive**
**Runcorn Cheshire(GB)**

(74) Representative: **Thomas, Ieuan et al,**
**Imperial Chemical Industries PLC Legal Department:**
**Patents PO Box 6**
**Welwyn Garden City Herts, AL7 1HD(GB)**

(54) **Microbial transformations.**

(57) Microbial transformations may be carried out in a bed of one or more micro-organisms supported in the pores of the particles of a porous particulate support, which particles comprise or contain one or more substrates for the micro-organism and which bed is substantially free of a continuous liquid phase.

*Fig. 1.*

EP 0 137 688 A2

QM 32855 \Ef·

## MICROBIAL TRANSFORMATIONS

This invention relates to devices in which microbial transformations may be carried out, to processes carried out therein and to certain of the products of such microbial transformations.

By "microbial transformation" we mean the conversion of a first carbon-containing compound (hereinafter referred to for convenience as "the substrate") into a second carbon-containing compound by a micro-organism, the substrate may be inorganic or organic and the second carbon-containing compound is organic; the second carbon-containing compound may be further metabolised by the micro-organism.

As examples of microbial transformations we would mention <u>inter alia</u> growth of micro-organisms, e.g. in the production of so-called single cell protein; the production by micro-organisms of (1) primary metabolic products, i.e. compounds which are essential for growth of the micro-organism, e.g. lysine, and (2) secondary metabolic products, i.e. compounds which are not essential for growth of the micro-organism, e.g. antibiotics; and replication of intruder molecules, e.g. viruses, in micro-organisms.

In the production of, for example, single cell protein, microbial transformation is often carried out in the presence of a large volume of water, typically the dry solids content of the reaction mixture used in the process is less than a few weight per-cent, to maintain the viscosity of the reaction mixture at a workable level. The product then has to be separated from the large volume of water. Where single cell protein is grown as a bacterium, the cells, because of their small size, often have to be agglomerated, e.g. flocculated, before they can be recovered, for example by centrifugation. Such recovery is expensive.

Recently, it has been proposed in German Patent Specification No. 2845387 that single cell protein may be produced by growth of yeast particles which are continuously fed with a spray of nutrient in a so-called "solid state fermenter", thus alleviating the problems associated with recovery of products from large volumes of aqueous media.

In US 4046921 it has been proposed that a koji for use in enzymatic preparations may be produced by growth of micro-organisms on certain solid culture media which are in the form of fluidised beds supplied with a liquid in an atomised state.

We have now found that microbial transformations can be effected in a bed of micro-organisms which are located in the pores of the particles of a suitable porous particulate support material which bed is substantially free of a continuous liquid phase. Transformation occurs substantially uniformly throughout the bed and the concentration of desired product, expressed as weight per unit volume of reactor, is often many times the concentration of the same product obtained from the same micro-organism in a conventional liquid phase reactor. Growth of the micro-organism within the pores of the porous particulate support alleviates damage which might occur to micro-organisms attached to the surface of particles which are in a fluidised bed. Furthermore, where the microbial transformation produces single cell protein, it is possible, by careful selection of a suitable particulate support, to produce a product, including the particulate support, which may be used as a human food or animal feedstock.

According to the present invention there is provided a device for effecting microbial transformation comprising a bed of one or more micro-organisms which are located in the pores of the particles of a suitable porous particulate support material, which particles comprise or contain one or more substrates, and which bed is substantially free of a continuous liquid phase.

The porous particles used in the present invention have a porosity of at least 20%.

The size of the pores and the numbers thereof in the particles depend inter alia on the nature of the micro-organism which is located in the pores and the microbial transformation which is effected by the micro-organism. Simple experiment will readily reveal a suitable combination of pore size and pore number.

Beds of which devices according to the present invention are comprised may be stationary beds, or agitated beds in which the particles of which the bed is comprised move relative to each other, e.g. in an Archimedian screw, in a rotating chamber, or in a fluidised bed, e.g. a vibration or spouted bed, or a homogeneous fluidised bed. The type of bed used may be chosen in the light of the micro-organism which is to be used in the device. For example, where the micro-organism grows slowly it is often preferred that a stationary bed is used; where the micro-organism grows rapidly it is often preferred that a homogenous fluidised bed is used.

By "fluidised bed" we mean a mass of particles through which a gas is passed substantially uniformly upward at a velocity sufficient to counteract the weight of the particles and hold them in a suspended apparently weightless condition. The passage of the gas causes agitation of the suspended particles and the mass resembles a boiling liquid.

Micro-organisms used in devices according to the present invention may be moulds, yeasts, bacteria, actinomycetes, algae or fungi. They may be aerobic, anaerobic, or facultative (i.e. the micro-organism can switch its metabolic machinery from aerobic to anaerobic or vice versa); they may be autotrophic or heterotrophic. As examples of suitable micro-organisms may be mentioned inter alia bacteria, for example, pseudomonas sp, e.g. P.putida; methylotrophs, e.g. M.methylotrophus; Bacillus, streptomyces sp, e.g. Streptomyces verticullum, Streptomyces laverlulae and arthrobacter; fungi, e.g. Aspergillus niger; and yeasts, e.g. Kluyveromyces fragilis.

A plurality of micro-organisms may be used in devices according to the present invention, which may be from different species or genera. Where a plurality of micro-organisms are employed symbiosis may occur.

The material of which porous particulate supports used in devices according to the present invention are composed may be inorganic, e.g. porous glass, diatomaceous earths, vermiculite prills, sintered ceramic or metallic foam. Preferably, however, the material is organic, e.g. a synthetic material such as a polyamide, polyester or ion-exchange resins, and more preferably it is naturally occurring e.g. starch, cellulose, polyhydroxy butyrate or a protein. Where it is desired to produce a secondary metabolite in the device the material is preferably metabolised by the micro-organism and more preferably it is a proteinaceous plant material, e.g. so-called "cotton seed media".

The porous particulate support may serve as a source or reservoir of nutritional material required for the microbial transformation occurring in the devices of the present invention. For example, where the particulate support material comprises cellulose or a protein it may provide energy, carbon or nitrogen for the microbial transformation. Where the particulate support material comprises, for example, polyvinlypyrrolidone nutrients may be slowly released therefrom, preferably at a controlled rate, which nutrients are required for the microbial transformation.

In a first preferred embodiment of the present invention, the material of which the porous particulate support is comprised is an organic material which is metabolisable by the micro-organism and the particles have a porosity of 30-50%.

In a second preferred embodiment of the present invention, the material of which the porous particulate support is comprised is an inorganic material which is not metabolisable by the micro-organism and the particles have a porosity of 75-99%. In such devices, it is often necessary to provide a gaseous or liquid source of carbon.

The particles of porous particulate support material used in devices according to the present invention may have a variety of shapes, e.g. cubic, pyramidal, disc, and spherical is often preferred. However, the shape used is often chosen in the light of the particular microbial transformation which is to be carried out.

The size of the particles of the porous particulate support material is in the range 10 microns to 10 mm, preferably in the range 100 microns to 5 mm and more preferably in the range 500 to 1200 microns. Where the particles are filamentous, particularly where they are filamentous organisms, the diameter of the filaments is in the range 0.05 microns to 10 microns and preferably in the range 0.1 microns to 2 microns.

In devices according to the present invention, it is essential that the micro-organisms are in an environment containing sufficient water to support microbial activity. The aforementioned water may serve as a reservoir for the substrate and the nutrients. It is preferred that the extra-cellular water content of the bed is in the range 1% w/w up to about 60% w/w of the wet bed and preferably is in the range 10-30% w/w and more preferably is about 15% w/w. The concentration of extra-cellular water in the bed may be maintained at the desired level by adding water thereto in the gaseous phase. For example, air saturated with water vapour may be passed through the bed.

Where living cells are used in devices according to the present invention it will be appreciated that the nutrients and other substances needed to maintain the viability of the cells must be provided. With regard to nutritional needs, a primary need is a source of energy for the cells metabolism. Often the energy source is a carbon-containing compound which is also the substrate for the particular microbial transformation which is occurring in a device according to the present invention, e.g. as in the conversion of sugar into ethanol.

After carbon, the nutrients needed in substantial quantities for microbial transformation are nitrogen and phosphorus. Other nutrients, such as vitamins and metal ions, are required in smaller amounts.

Where oxygen is required in devices according to the present invention air or oxygen-enriched air may be used.

Whilst we do not exclude the possibility that the nutritional requirements of micro-organisms used in devices according to the present invention may be added to the bed in liquid form, e.g. in the form of a spray of droplets of a neat liquid or solution, often an aqueous solution, preferably at least a portion, and more preferably at least a major portion, of a nutritional requirement, e.g. an energy source, is provided in or by the gas phase. Where a fluidised bed is used at least a portion of the aforesaid nutritional requirement may be provided in or by the fluidising gas. It will be appreciated that where the gas is recycled it will be necessary to monitor the composition thereof such that the desired concentration of the component gases thereof may be maintained. For example, carbon dioxide is produced by the micro-organism and the concentration thereof in the gas may rise to levels which have a deleterious effect on the micro-organism.

Where a nutrient or energy source for use in devices according to the present invention is gaseous, e.g. carbon dioxide, hydrogen sulphide or a hydrocarbon, e.g. methane, or a readily volatised liquid, e.g. methanol or ethanol, it may be fed to the bed in or with the gaseous phase. For example, where the nutrient or energy source is a readily volatised liquid, the fluidising gas may be passed through a reservoir of the readily volatilised liquid. Where a nutrient or energy source is a liquid, e.g. gas-oil, it may be sprayed onto the fluidised bed as a neat liquid or mixed with a suitable liquid diluent, e.g. a solvent therefor. Where a nutrient or energy source is a solid, e.g. glucose, it may be sprayed, as a solution in a solvent therefor, onto the fluidised bed. It will be appreciated that where a liquid diluent or solvent is used it will be chosen such that it does not adversely affect the bed of micro-organisms, or any product produced therefrom.

It will be appreciated that the temperature at which a microbial transformation is carried out in devices according to the present invention often has to be controlled within a narrow range. However, we do not exclude the possibility that thermophilic micro-organisms may be employed.

Where a filamentous micro-organism is used in devices according to the present invention it may be a fungi, algae, or bacterium, e.g. a blue-green algae or an actinomycetales. As examples of actinomycotales we may mention _inter alia_ streptomyces, micromonospora, streptosporangium, and actinomycetes.

The micro-organisms used in the present invention may be encapsulated in semipermeable microcapsules, typically having mean diameters of less than 15 microns. Methods for preparing microcapsules are more fully described in Science, 1964, Volume 146, pages 524-527.

Where living cells are used in the process of the present invention we do not exclude the possibility that mutation of at least a portion of the cells may occur.

Where single cell protein is produced in devices according to the present invention the porous particulate support material may be removed from the product, for example by dissolution in a suitable solvent. Preferably, however, the porous particulate support material is retained in the product, more preferably as a nutritional additive when the product is used as human food or animal feedstock.

Inclusion of appropriate additives in the porous particulate support material often affords increased control of the microbial transformation. For example, the following additives may be present in the particulate support: nutrients, buffering agents, means for controlling pH, e.g. calcium carbonate, phosphate buffer; trace elements; e.g. magnesium; enzymes and/or micro-organisms for removing toxic by-products.

A preferred particulate support material is formed by aggregating small particles, e.g. 5-35 microns, of organic material, and particles of a second material, for example a buffer, e.g. calcium carbonate, capable of reacting with a by-product of the microbial transformation which has an adverse effect on the microbial transformation, e.g. an acid, in the presence of an aqueous carbohydrate solution, e.g. starch, such that the particles are bound together by the carbohydrate to form particles of a size suitable for use as a particulate support material in devices according to the present invention. The weight ratio of the original material to the second material is typically between 10:1 and 1:2.

It will be appreciated that the maintenance of strict aspectic conditions is often essential to avoid deleterious contamination of the culture used in devices according to the present invention by foreign micro-organisms. Such contamination may, for example, inhibit or interfere with cell or enzyme activity, or even destroy it, or produce noxious by-products.

It will be appreciated that the nature of the substrate used in a particular device according to the present invention may affect the design of the device and its associated systems. For example, hydrocarbons and fats contain less oxygen than carbohydrates and on changing the substrate from a carbohydrate to a hydrocarbon or fat more oxygen, often up to three times more, has to be provided and the heat released on consumption of the substrate is increased by a similar ratio.

Where devices according to the present invention comprise a fluidised bed the pressure and quantity of the fluidising gas used therein will depend inter alia on the density and volume of the bed and on the amount of heat which is generated during the microbial transformation.

Whilst at least a portion of the heat generated by microbial transformations which are carried out in devices according to the present invention may be lost by evaporation, or removed by the fluidising gas, where a fluidised bed is used, it is often preferred that heat-absorption means, e.g. cooling plates or tubes, are suitably disposed in the device for removal of a portion of the heat.

Where a fluidised bed is used in devices according to the present invention it may be operated in a segregating mode in which "older" particles sink to the bottom of the reactor and are removed or in a mixed mode with a separate elutriator and recycle of "younger" particles.

It will be appreciated that devices according to the present invention are provided with appropriate means for charging the bed with whatever reagents are required for carrying out the microbial transformation occurring therein and for recovering the desired product therefrom.

Typically, the device is provided with suitable ports for the entry and exit of gaseous phases and ports for charging and discharging liquid phases and solid phases.

Products which may be prepared in devices according to the present invention include antibiotics, enzymes, organic acids, e.g. acetic acid, citric acid, glutamic acid, lysine, drugs, e.g. cortisone, hydrocortisone, vitamins, polysaccharides, purine nucleotides, fructose syrups, proteases, and so-called single cell protein for human food and feedstock.

Where a product of a microbial transformation which is carried out in a device according to the present invention is a volatile liquid the liquid may be removed in the fluidising gas, where a fluidising gas is used and collected in, for example, a suitable condenser.

Where the product of a microbial transformation which is carried out in a device according to the present invention is not readily volatilised then the support and the micro-organism may be subjected to an appropriate treatment, e.g. solvent extraction, to remove the product.

In a preferred aspect of the present invention, the porous particulate support material is an edible material on which a micro-organism suitable for the production of single cell protein, preferably a strain of Methylophilus methylotrophus, is grown. The product, including the support may be used directly, or after addition of various additives, as an animal feedstock.

The present invention is illustrated by reference to the accompanying drawing which shows, by way of example only, one embodiment of a device according to the present invention.

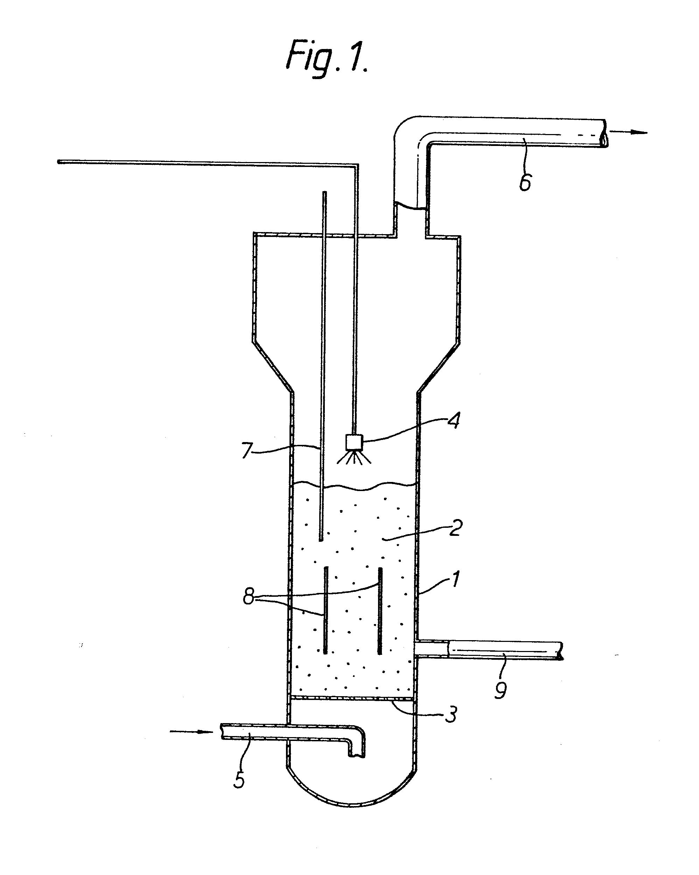

Figure 1 is a diagrammatic representation of a device according to the present invention.

In Figure 1, a reactor 1 contains a bed 2 of a micro-organism located in the pores of a porous particulate support material, which bed is carried on a suintered stainless steel plate 3. The reactor 1 is provided with a two-phase spray nozzle 4, a gas inlet pipe 5, a gas discharge pipe 6, monitoring probes 7, heat transfer means 8, e.g. cooling tubes, and product discharge pipe 9.

In operation, a bed 2 of micro-organisms located in the pores of a porous particulate support material is fluidised by continuous supply of air which flows through inlet pipe 5 and sintered plate 3 and is discharged via gas discharge pipe 6. A supply of essential nutrients for the micro-organism is provided via spray nozzle 4 while the conditions in the bed are monitored by probes 9 and the temperature in the bed is maintained at a desired temperature by heat transfer means 8. When desired, the bed is discharged through pipe 9.

The present invention is illustrated by the following examples.

Solutions of Trace Elements

Aqueous solutions (Solution A and Solution B) were prepared which contained a variety of trace elements. The trace elements were present in the compounds shown in Tables 1 and 2 which Tables also show the concentrations of the compounds in the solutions.

TABLE 1

| Compound | Concentration (grams/litre) |
|---|---|
| Ferrous sulphate heptahydrate | 1 |
| Zinc sulphate heptahydrate | 1 |
| Copper sulphate pentahydrate | 0.15 |
| Potassium molybdate | 0.1 |
| Manganese sulphate heptahydrate | 0.1 |

TABLE 2

| Compound | Concentration (grams/litre) |
|---|---|
| Ferric chloride | 0.076 |
| Copper sulphate pentahydrate | 0.005 |
| Manganese sulphate tetrahydrate | 0.026 |
| Zinc sulphate heptahydrate | 0.026 |
| Calcium carbonate | 0.5 |
| Magnesium sulphate heptahydrate | 6.6 |
| Ammonium sulphate | 59 |

Examples 1-8

General Procedure

A porous particulate support material was prepared by granulating a proteinoceous plant medium, e.g. cotton seed medium, and one or more other materials with a 5% w/w soluble starch solution. The granulated product was dried in a fluidised bed and a portion of the porous particulate support material, screened to a particle size of 600-850 microns (except Example 3 where the particle size was 600-825 microns), was charged to a cylindrical vessel of internal diameter 32 mm and height 40 mm.

A bed was formed by inocculating the porous particulate support material in the cylindrical vessel with a suspension (5 ml) of a micro-organism; the suspension contained 6 ml (except Examples 3 and 7 which contained 60 ml and 40 ml respectively) of a trace element solution per litre of suspension. (Example 8 contained in addition 1 ml of a 0.1% w/v ferric chloride solution per litre of suspension).

Air saturated with water vapour at a desired temperature was passed through a sintered glass filter in the base of the cylindrical vessel to aerate the bed. In Example 3, a second air stream, containing equimolar proportions of ethanol and toluene vapour, at a rate of 25 ml per minute was also used. In Example 8, the air was passed through a 1.5% v/v solution of methanol in water at 36°C prior to aerating the bed.

Details of the beds and the operating conditions are shown in Tables 3 and 4 respectively. In Table 4, the column headed "Growth of Micro-organism" shows the increase in cell numbers during the indicated period of time, calculated from the carbon dioxide concentration in the gas discharged from the bed.

In Example 1, ethanol was produced at a rate of 4.3 grams/litre of bed over the 162 hours reaction time.

In Example 3, cis-2,3-dihydroxy-toluene was produced at a rate of 27 grams/litre of bed over the 24 hours reaction time.

Example 9

A porous particulate support material was prepared by granulating cotton seed medium (400 grams), 10% soluble dextrin (50 grams), and calcium carbonate (50 grams) with a 5% w/w soluble starch solution (500 grams).

## TABLE 3

| Ex-ample No. | Weight Ratios used in the Preparation of the Porous Particulate Support | | | | | Micro-organism | Minor Element solution |
|---|---|---|---|---|---|---|---|
| | Cotton Seed Mediuma | Lac-tose | 10% Soluble Dextrinb | Calcium Carbon-atec | 5% w/w Soluble Starch | | |
| 1 | 8 | 1 | 0 | 1 | 10 | Kluyver-omyces fragilis ATCC28244 | A |
| 2 | 8 | 0 | 1 | 1 | 10 | Arthro-bacter Sp | A |
| 3 | 8 | 0 | 2 | 0 | 10 | Pseudomo-nas putida NCIB 1767 | Fison's |
| 4 | 1 | 0 | 8 | 1 | 10 | Strepto-myces longispor-oflavus | A |
| 5 | 1 | 0 | 8 | 1 | 10 | Strepto-myces verti-cillium | A |
| 6 | 8 | 0 | 1 | 1 | 10 | Alcali-genes eutrophus | A |
| 7 | 2 | 0 | 8 | 0 | 10 | Asper-gillus niger ATCC22343 | B |
| 8 | 1 | 0 | 8 | 1 | 10 | Methylo-philus Methylo-trophus NCIB10592 | A |

a: Mean particle size 25 microns  b: Mean particle size 17 microns

c: Mean particle size 43 microns

## TABLE 4

| Example No. | Rate of Air flow (ml/min) | Temperature of Air (°C) | Reaction Time (hours) | Extra-cellular water content (%) | Growth of Microorganism |
|---|---|---|---|---|---|
| 1 | 100 | 28 | 162 | 51 | 6x/10 hours |
| 2 | 100 | 30 | 76 | 42 | 40x/11 hours |
| 3 | 43 | 28 | 28 | 26 | a |
| 4 | 100 | 28 | 28 | 55 | 100x/160 hours |
| 5 | 100 | 28 | 28 | 42 | 12x/116 hours |
| 6 | 100 | 36 | 36 | 40 | 77x/51 hours |
| 7 | 100 | 28 | 28 | 55 | 284x/74 hours |
| 8 | 100 | - | - | 33 | 10x/9 hours |

a: Not determined.

The granulated product was dried in a fluidised bed and a portion (800 grams) of the dried product, screened to a particle size of 600-1180 microns, was charged to a cylindrical vessel of internal diameter 100 mm and height 400 mm. The porous particulate support material was fluidised with a stream of air which was humidified to a relative humidity of 26% at 49°C. The humidified air was introduced, at a superficial velocity of 42 cm/ second, through a sintered stainless steel plate in the base of the cylindrical vessel. The porous particulate support material was moistened to a level of 15% w/w by spraying water onto it at a rate of 36 ml/hour via a two phase atomising spray nozzle. The reactor was stabilised at a temperature of 28°C with the relative humidity of the discharged gas being 90% at 28°C. A suspension (200 ml) of Streptomyces longisporoflavus was charged to the porous particulate support material at a rate of 36 ml/hour to form a fluidised bed. Water, containing 1 ml of Solution A per litre of water, was charged to the bed at 36 ml/hour for 177 hours. During this period there was a ten-fold increase in the concentration of cells as indicated by the carbon dioxide concentration in the discharged phase.

## CLAIMS

1.    A device for effecting microbial transformation comprising a bed of one or more micro-organisms which are located in the pores of the particles of a suitable porous particulate support material which particles comprise or contain one or more substrates the said bed being substantially free of a continuous liquid phase.

2.    A device as claimed in claim 1 wherein the porosity of the particles is between 30 and 50%.

3.    A device as claimed in claim 1 wherein the porosity of the particles is between 75 and 99%.

4.    A device as claimed in claim 1 in which the micro-organism is a bacterium, fungus or yeast.

5.    A device as claimed in claim 4 in which the bacterium is selected from the group consisting of Pseudomonas putida, Methylophilus methylotrophus, Streptomyces verticullum, Streptomyces laverlulae, Arthrobacter, Aspergillus niger, Kluyveromyces fragilis, and Streptomyces longisporoflavus.

6.    A device as claimed in claim 1 in which the porous particulate support material comprises at least one substrate.

7.    A device as claimed in claim 1 in which the porous particulate support material is edible.

8.    A device as claimed in claim 1 in which the particles are less than 5 mms.

9.    A device as claimed in claim 8 in which the particles are between 500 and 1200 microns.

10.    A device as claimed in claim 1 in which the extra-cellular water content of the wet bed is in the range 1-60% w/w.

11.    A device as claimed in claim 10 in which the extra-cellular water content of the wet bed is about 15% w/w.

12.    A process for effecting microbial transformation whenever carried out in a device as claimed in claim 1.

## Fig.1.